Europäisches Patentamt

European Patent Office

Office européen des brevets

(19)

(11) Publication number: **0 097 907**
A2

## (12) EUROPEAN PATENT APPLICATION

(21) Application number: **83106050.4**

(22) Date of filing: **21.06.83**

(51) Int. Cl.³: **B 01 J 13/00**
**C 12 N 5/02, C 12 P 21/02**
**B 01 J 2/02**

(30) Priority: **25.06.82 US 392218**

(43) Date of publication of application:
**11.01.84 Bulletin 84/2**

(84) Designated Contracting States:
**BE DE FR GB IT NL SE**

(71) Applicant: **FLOW GENERAL, INC.**
**7655 Old Springhouse Road**
**Mclean Virginia 22102(US)**

(72) Inventor: **Haller, Wolfgang K.**
**4620 N. Park Avenue Apt. 909E**
**Chevy Chase, MD(US)**

(74) Representative: **Patentanwälte Grünecker, Dr.**
**Kinkeldey, Dr. Stockmair, Dr. Schumann, Jakob, Dr.**
**Bezold, Meister, Hilgers, Dr. Meyer-Plath**
**Maximilianstrasse 58**
**D-8000 München 22(DE)**

(54) **Cell culture microcarriers.**

(57) Cell culture microcarriers made of water-insolubilized protein. The protein is substantially homogeneously distributed throughout the microcarrier. The cell culture microcarrier is made by forming droplets of an aqueous solution of a protein, suspending the droplets in a fluid, solidifying the suspended droplets into cell culture microcarriers and recovering the cell culture microcarriers. Preferably, prior to recovery, the cell culture microcarriers are hardened with a crosslinking agent, preferably glutaraldehyde and neutralized with a dilute solution of the protein. An apparatus for converting a water-based protein solution to microspheres. The apparatus has a heated, thermostatically controlled reservoir for the protein solution. The protein solution is supplied to a spray head. The protein solution is maintained at approximately at least the same temperature at the spray head as in the reservoir and a predetermined pressure is maintained on the protein solution at the spray head. A receptacle receives the droplets emitted by the spray head. The droplets are suspended and solidified into microspheres by flowing a low temperature, water immiscible suspension liquid across the receptacle and microspheres are collected. The cell culture microcarriers have been used to grow and harvest anchorage dependent cells and anchorage dependent cell products, such as interferon. The cell culture microcarriers produce outstanding cell growth and possess superior subcultivation ability.

FIG. 1

EP 0 097 907 A2

GRÜNECKER, KINKELDEY, STOCKMAIR & PARTNER

PATENTANWALTE
EUROPEAN PATENT ATTORNEYS

A GRUNECKER, DRL ING
DR H KINKELDEY, DRL ING
DR W STOCKMAIR, DRL ING, AE E ICALTECH
DR K. SCHUMANN, DRL PHYS
P. H. JAKOB, DRL ING
DR G BEZOLD, DRL CHEM
W MEISTER, DRL ING
H. HILGERS, DRL ING
DR H. MEYER-PLATH, DRL ING

8000 MÜNCHEN 22
MAXIMILIANSTRASSE 58

# CELL CULTURE MICROCARRIERS

This invention relates to cell culture microcarriers. The invention also relates to methods of growing anchorage dependent cells and of producing both anchorage dependent cells and anchorage dependent cell products. The invention further relates to an apparatus for converting a water-based protein solution to microspheres.

The _in vitro_ culture of cells is an important research tool, as well as an industrially useful process. Specifically, it is important to produce cells and also to collect such useful cell products as viral vaccines, hormones and other substances, including interferons.

Cells that are not anchorage-dependent will grow freely when suspended in a suitable medium. For such cells, scaling up from the research laboratory to an industrial operation is not excessively difficult, particularly because the cultures are essentially homogeneous. Consequently, the cells and the medium can be readily sampled for examination. The medium surrounding the cells can also be readily removed, replaced or otherwise modified.

In contrast, large scale culture of anchorage dependent cells has involved various difficulties. Traditionally, such cells were grown on the inside surface of glass or plastic vessels, such as bottles and dishes. In large scale applications, these traditional growth techniques impose severe restrictions because of limited growth surfaces.

Moreover, such traditional techniques tend to waste growth medium because the growth-area to medium-volume ratio of containers such as bottles and dishes, cannot be increased above a certain value. Because the medium cannot be readily sampled, it is difficult to determine the properties of both the medium and the cells during growth. Further, the medium composition cannot be easily adjusted.

Improvements in the growth-area to volume ratio, as well as in the ability to adjust the sample medium, were provided by techniques wherein cells were grown on the surface of stationary beds of solids, such as ceramic rings and glass tube segments. In such techniques, the medium is circulated through the stationary bed, allowing the medium to be monitored and adjusted at another point of the circulation loop. Unfortunately, such perfusive, stationary-supported cell culture growing methods do not provide cells that are accessible for sampling. Disadvantageously, moreover, the stationary beds may become irreversibly clogged, leading to progressive cell death and further clogging as parts of the bed become inaccessible to the life-supporting circulation medium. The problem of clogging becomes particularly severe if attempts are made to improve the growth-area to volume ratio by reducing the size of the solid bodies in the bed.

The practice of growing anchorage-dependent cells advanced remarkably with the introduction of the stirred microcarrier technique, which is designed to extend all the advantages of homogeneous, suspended cell cultures to anchorage-dependent cells. In principle, cells are grown on small carrier particles, also known as carrier bodies or cell culture microcarriers. These cell culture microcarriers have the requisite shape, size and density to be suspended and fluidized in an agitated growth medium. The cell culture microcarriers also have surface properties suitable for supporting cell growth. See Van Wezel, 216 Nature 64-65 (1967).

In particular, Van Wezel demonstrated that cells could attach to the positively charged surfaces of a commercially available dextran-based anion exchanger. The use of cell culture microcarriers was slowed, however, by the nonspecific toxicity of the microcarriers to cells. Examining the causes of this problem, Thilly and his coworkers found that a reduction in the number of positive charges on the ion exchange bead also reduced toxicity problems with positively charged dextran-based microcarriers. See Levine et al., Somatic Cell Genetics 3, 149 (1977); and Levine et al., U.S. Patents 4,189,534 and 4,293,654. Other positively charged microcarrier beads, based upon polyacrylamide, are described by Monthony et al. in U.S. Patent 4,237,218.

Such positively charged ion-exchanger type beads, although successfully used in microcarriers, possess a significant disadvantage. Because of the electrostatic interaction between the normally negatively charged cells and the positively charged microcarriers, it is difficult to remove the cells from the microcarriers without reducing the viability of a significant fraction of the cells. Consequently, the harvesting of cells or of cell-associated products and the subcultivation of cells onto additional microcarrier beads have both continued to be difficult and troublesome operations. Accordingly, workers in the art have sought improved cell culture microcarriers for obtaining improved methods of producing both anchorage dependent cells and anchorage dependent cell products.

The present invention overcomes the problems and disadvantages of the prior art by producing cell culture microcarriers which have surface structures conducive to cell attachment and growth of cells. The cell culture microcarriers of the present invention do not exhibit electrostatic interaction which interferes with cell removal for harvesting or subcultivation purposes. Moreover, it has been discovered that the cell culture microcarriers of the present invention are not toxic to cells. Therefore, an object of the present invention is to produce cell culture microcarriers having a surface structure conducive to cell attachment and growth of cells without electrostatic interferences with cell removal for harvesting or subcultivation purposes.

It is another object of the invention to produce cell culture microcarriers which are not toxic to cells.

It is a further object of the invention to produce a cell culture microcarrier in a single process without the necessity of grafting a material to the surface of the cell culture microcarriers.

It is a further object of the invention to produce cell culture microcarriers at a reasonable cost by a method in which no grafting is needed, in which the cell culture microcarrier beads remain hot-water soluble throughout most of the collection process, in which cleaning is easy and hygienic, in which there are no problems of pot life associated with the material from which the cell culture microcarriers are made, in which microcarriers outside a certain size range can be recycled and in which cell culture microcarriers having a high size uniformity are obtained.

It is a further object of the invention to provide cell culture microcarriers which can be used repeatedly in the growth of anchorage-dependent cells.

It is a further object of the invention to provide cell culture microcarriers from which attached anchorage-dependent cells can be separated by mild treatment with proteolytic agents, such as trypsin, or other agents, such as EDTA.

Additional objects and advantages of the invention will be set forth in part in the description which follows and in part will be obvious from the description or may be learned by practice of the invention. The objects and advantages of the invention may be realized and attained by means of the instrumentalities and combinations particularly pointed out in the appended claims.

To achieve the objects and in accordance with the purpose of the invention, as embodied and broadly described herein, the cell culture microcarrier according to the present invention comprises a water-insolubilized protein, the protein being substantially homogeneously distributed throughout the microcarrier.

Further, to achieve the foregoing objects and in accordance with the purpose of the invention, as embodied and broadly described herein, the method for producing cell culture microcarriers according to the present invention comprises the steps of:

(a)    forming droplets of a solution of a protein;

(b)    suspending the droplets in a fluid;

(c)    solidifying the suspended droplets into cell culture microcarriers; and

(d)    recovering the cell culture microcarriers.

Still further, to achieve the foregoing objects and in accordance with the purposes of the invention, as embodied and described herein, the method of growing anchorage dependent cells according to the present invention comprises the steps of:

(a)  providing a suspension comprising cell culture microcarriers, an innoculum of the cells and a nutrient-containing growth medium, the microcarriers comprising a water-insolubilized protein, the protein being substantially homogeneously distributed throughout the microcarrier; and

(b)  maintaining the suspension under conditions conducive to cell growth.

Still further to achieve the foregoing objects and in accordance with the purpose of the invention, as embodied and broadly described herein, the method of producing anchorage dependent cells according to the invention comprises the steps of:

(a)  providing a suspension comprising cell culture microcarriers, an  innoculum of the cells and a nutrient-containing growth medium, the cell microcarriers comprising a water-insolubilized protein, the protein being substantially homogeneously distributed throughout the microcarriers;

(b)  maintaining the suspension under conditions conducive to cell growth;  and

(c)  harvesting the cells.

Still further to achieve the foregoing objects and in accordance with the purposes of the invention, as embodied and broadly described herein, the method of producing anchorage-dependent cell products according to the present invention comprises the steps of:

(a)  forming a suspension, in a suitable cell culture medium, of cell culture microcarriers comprising a water-insolubilized protein, the protein being substantially homogeneously distributed throughout the microcarrier;

(b)  inoculating the culture with anchorage-dependent cells to form a cell culture;

(c)    maintaining the cell culture under conditions conducive  to production of cell products;  and

(d)    harvesting the cell products.

Still further to achieve the foregoing objects and in accordance with the purpose of the invention, as embodied and broadly described herein, an apparatus for converting a water-based protein solution to microspheres according to the present invention comprises;

a heated, thermostatically controlled reservoir for the protein solution;

at least one spray head;

means for supplying the protein solution from the reservoir to the spray head;

means for applying heat to the supplying means for maintaining the protein solution at approximately at least the same temperature as the protein solution in the reservoir;

means for maintaining a predetermined pressure on the protein solution at the spray head;

at least one receptacle positioned beneath the spray head for receiving the droplets emitted by the spray head;

means for flowing a low temperature, water immiscible suspension liquid across at least one receptacle for suspending and solidifying the received droplets into discrete microspheres in the suspension liquid;  and means for collecting the microspheres from the suspension liquid.

Still further to achieve the foregoing objects and in accordance with the purpose of the invention, as embodied and broadly described herein, a method for converting a water-based protein solution into microspheres according to the present invention comprises the steps of:

spraying the protein solution as a liquid into a gaseous
atmosphere to form discrete droplets;

maintaining a temperature differential between the solution
and the gaseous atmosphere wherein the gaseous atmosphere is at a lower temperature than the temperature of
the solution before spraying;

flowing a cooled water-immiscible suspension liquid across
the path of the fall of the discrete droplets to form
microspheres suspended in the cooled suspension liquid;
and

collecting the microspheres from the suspension liquid.


The accompanying drawings, which are incorporated in and
constitute a part of this Specification, illustrate embodiments of the invention and together with the description, serve to explain the principles of the invention.


Fig. 1 shows a perspective view of a preferred apparatus
for producing microspheres in accordance with the present
invention.

Fig. 2 shows a cross-sectional view of a receptacle for
receiving droplets as shown in Fig. 1.

Fig. 3 shows a cross-sectional view of a spray head,
as shown in Fig. 1.

Fig. 4 shows a bottom view of a whirl plate, as shown

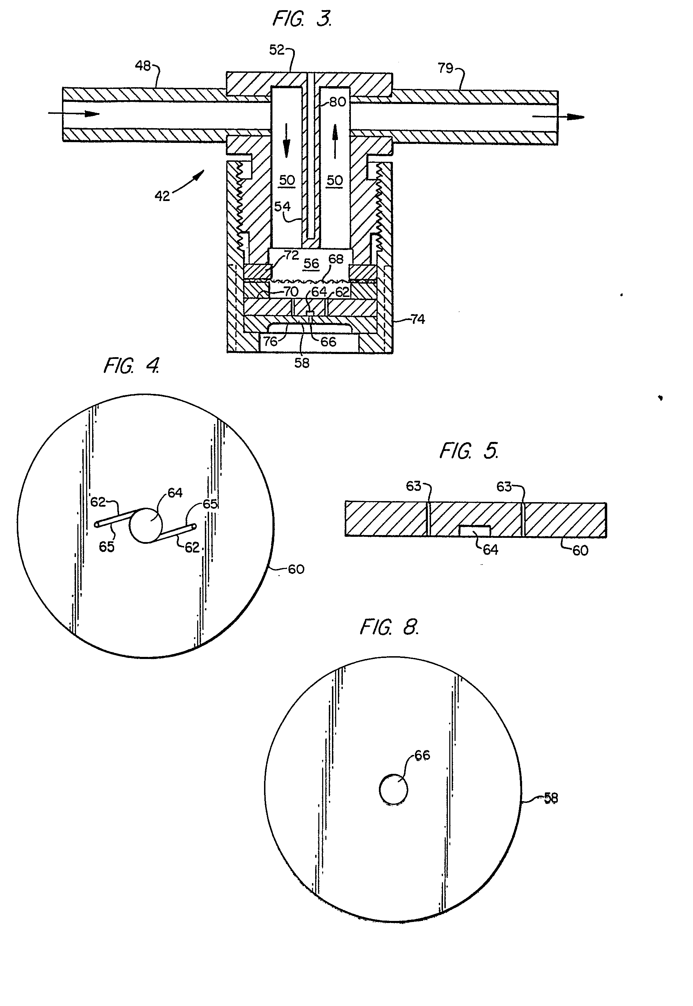

in Fig. 3.

Fig. 5 shows a cross-sectional view of the whirl plate
of Fig. 4.

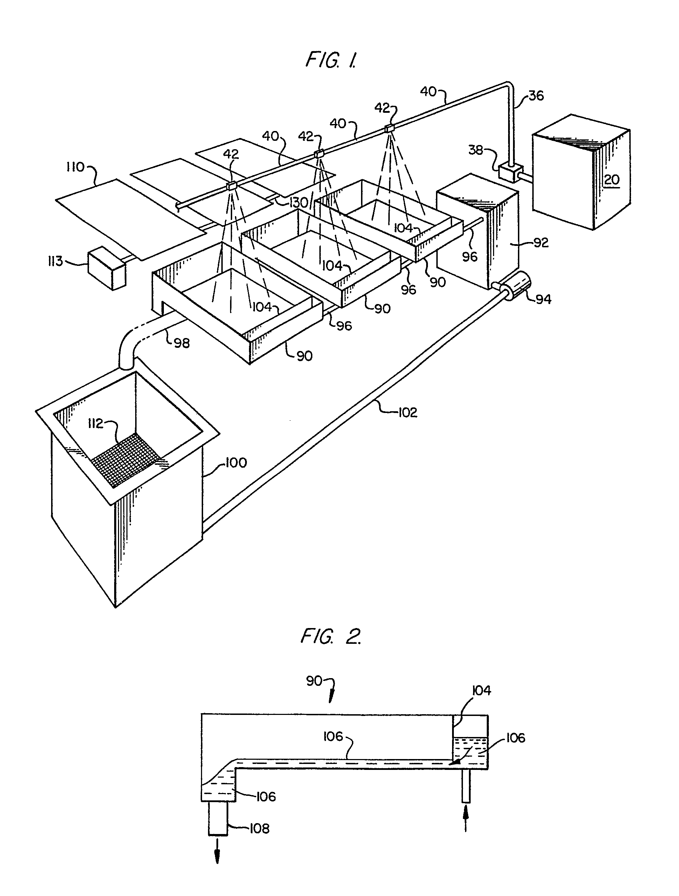

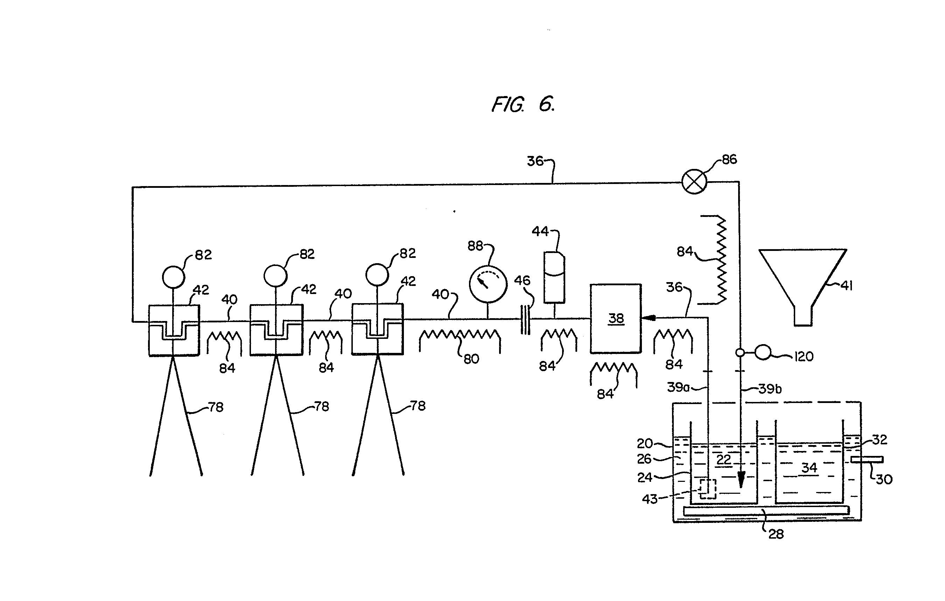

Fig. 6 is a schematic diagram of the apparatus of Fig. 1
in somewhat more detail.

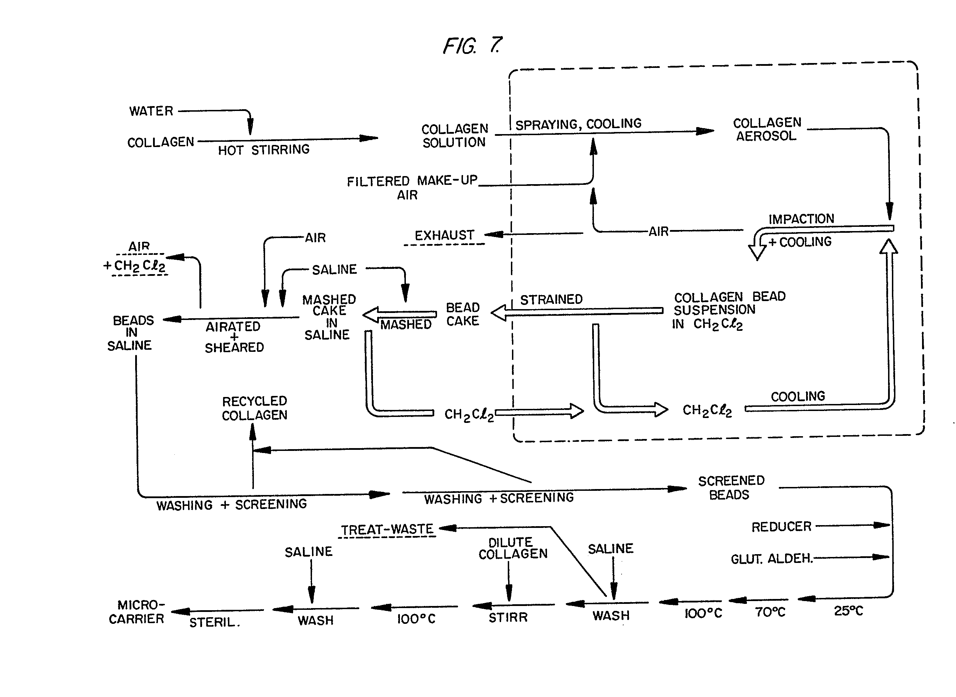

Fig. 7 is a flow chart showing a method for producing
cell culture microcarriers in accordance with the present
invention.

Fig. 8 shows a bottom view of a nozzle, as shown in
Fig. 3


Reference will now be made in detail to the presently
preferred embodiments of the invention, examples of which are illustrated in the accompanying drawings.

In accordance with the invention, cell culture microcarriers comprise a water-insolubilized protein. Preferred proteins include collagen, casein, soybean protein, silk, keratin, egg proteins and blood proteins. More preferably, the protein is collagen.

The microcarrier may comprise the reaction product of a protein and a cross-linking agent. Preferably, the cross-linking agent is multifunctional. Suitable multifunctional cross-linking agents contain such functional groups as aldehydes, epoxies, haloalkyls, haloaryls, isothiocyanates, isocyanates, diazo, arylnitrenes, azides, acylhalides, acylanhydrides, diolcarbonates, chlorofor-mates, imidoesters, N-hydroxysuccinimidylesters, malein-imides, carboxydiimidazols, formylesters and halotriazines. Preferably, the multifunctional cross-linking agent is glutaral-dehyde or 1,4,butanediol diglycidyl ether. Further preferred cross-linking agents include formaldehyde, epichlorohydrin, phosgene and aldehyde starch.

Preferably, the cell culture microcarriers have a diameter of about 50 to about 350 microns.

In accordance with the invention, droplets of an aqueous solution of a protein, such as described above, are formed. As is well-known, collagen may be liquified by heating and chemically degrading collagen to obtain an aqueous gelatin solution. The collagen may be obtained from pigskin via the well-known acid process. Alternatively, commercially available denatured collagen, such as gelatin type A, may be obtained and dissolved by heating in water.

To assure that the cell culture microcarriers obtained are uniform, well-known quality control tests such as viscosity, bloom rating, color, turbidity, isoelectric point and pH may be run on the collagen or other protein used. In order for the collagen to have sufficient mechanical strength, the bloom rating of the collagen should be from approximately 150 to approximately 300.

The droplets of the solution of the protein may be formed in various ways. Preferred ways of forming the droplets include atomizing the solution from a pressure nozzle, atomizing the solution from a gas-propelled spray nozzle, atomizing the solution from a rapidly spinning surface, atomizing the solution from an ultrasonically driven nozzle and emulsifying the solution. Suitable pressure nozzles, gas-propelled spray nozzles, rapidly spinning surfaces and ultrasonically driven nozzles are commercially available. Methods for emulsifying solutions of proteins are well-known.

In accordance with the invention, the droplets of the solution of the protein are suspended in a fluid. According to a preferred embodiment of the invention, the fluid, which may be a liquid medium or a gaseous medium, as explained in detail below, contains a solidification agent for the droplets. Preferred solidification agents include ammonia, an aldehyde, an epoxy compound and epichlorohydrin. Preferably, the aldehyde is a multifunctional aldehyde. More preferably, the multifunctional aldehyde is glutaraldehyde which is readily available commercially.

Preferably, the epoxy compound is a multifunctional epoxy compound. More preferably, the multifunctional epoxy compound is 1,4 butanediol diglycidyl ether, which is commercially available.

A particularly suitable aldehyde solidification agent for the droplets is formaldehyde. As is well-known, formaldehyde is commercially available.

In accordance with the invention, the fluid may be either a gaseous medium or a liquid medium. Preferred gaseous media include air, hydrogen chloride, ammonia, formaldehyde, glutaraldehyde, or combinations of these specific gaseous compounds. Preferably, the gaseous medium is maintained at a temperature lower than the droplets. More preferably, the droplets of solution are at a temperature of about 50°C and the gaseous medium is at room temperature or lower.

The liquid medium is preferably non-flammable and either immiscible with the droplets of the solution or miscible with the droplets. Preferably, the temperature of the liquid medium is maintained in a range from 0 to 10°C. However, the temperature of the liquid medium should not be below the freezing point of the protein solution because freezing of the droplets mechanically disrupts the microcarrier structure.

Preferred liquid media which are immiscible with the droplets are slightly soluble in water and rapidly evaporate below 50°C without leaving a residue. Specifically preferred immiscible liquid media are the halogenated hydrocarbons. Preferred halogenated hydrocarbons include methylene chloride ($CH_2Cl_2$). 1,1,1-trichloroethane, trichloroethylene and liquid trademarked Freon® products of DuPont de Nemours, E.I. & Co., which products contain fluorocarbons and/or chlorofluorocarbons, such as the well-known and readily available Freon® T.F.

Preferred liquid media miscible with the droplets include acids, bases and aldehydes. The acids may be inorganic or organic. Preferred inorganic acids include nitric acid, hydrochloric acid, phosphoric acid and sulfuric acid. Preferred organic acids include oxalic acid and formic acid. Preferred bases include ammonia, alkalihydroxide and amines.

Aldehydes are also suitable liquid media which are miscible with the droplets. Preferred aldehydes are formaldehyde

12

0097907

and glutaraldehyde.

In accordance with the invention, the suspended droplets are solidified into cell culture microcarriers. When solidified, colliding mircocarriers do not coalesce. Solidification may be accomplished by maintaining a temperature differential between the droplets of the solution of the protein and the fluid. Solidification may also result from a solidification agent for the protein which is present in the solution of the protein.

With respect to maintaining a temperature differential between the droplets of the solution of the protein and the fluid, the fluid may be hotter than the droplets of the solution or colder than the droplets of the solution. Further, the temperature of the fluid may be hotter than the temperature of the droplets of the solution and the solution may also contain an appropriate solidification agent, as described below. Further, to accomplish solidification, the fluid may be a gaseous medium, which may be maintained at a temperature lower than the droplets. The droplets, suspended in the gaseous medium may then be introduced into suspension in a liquid medium, which also may be maintained at a temperature lower than the droplets.

With respect to accomplishing solidification by the presence in the droplet of a solidification agent, it is, of course, required that the solidification agent be sufficiently slow acting that the protein solution does not solidify prior to being suspended as droplets in the fluid. The solidification agent is also water-soluble.

Preferred water-soluble solidification agents include aldehyde, acid and base. As described above, the aldehyde may be a multifunctional aldehyde, such as glutaraldehyde, or a monofunctional aldehyde, such as formaldehyde. The acid may be inorganic, such as nitric acid, hydrochloric acid, phosphoric acid and sulfuric acid, or organic, such as oxalic acid and formic acid. Preferred bases include ammonia, alkalihydroxide and amines.

In accordance with the invention, the cell culture microcarriers are recovered using either conventional techniques or those described in detail hereafter. In some instances, after solidifying the microcarriers but prior to recovering the microcarriers, it may be desired to further harden the cell culture microcarriers. Preferably, the hardening is accomplished by a cross-linking agent.

To accomplish hardening, the microcarriers may be suspended in a liquid or gaseous medium containing the cross-linking agent. Preferred cross-linking agents are multifunctional, including multifunctional aldehydes and multifunctional epoxies. Preferably, the multifunctional aldehyde is glutaraldehyde.

More preferably, the glutaraldehyde is combined with a reducing agent, preferably sodium dithionite. Addition of a reducing agent, such as sodium dithionite, to the glutaraldehyde produces cell culture microcarriers which are nearly colorless. Other preferred cross-linking agents include formaldehyde and 1,4 butanediol diglycidyl ether.

Preferably, the hardening effect of the cross-linking agent is further accelerated by the application of heat. More preferably, the hardening effect of the cross-linking agent is further accelerated by increasing the application of heat without liquifying the microcarriers. To achieve this, the microcarriers are subjected to a temperature ramp during which only occasional stirring is needed.

In a particularly preferred embodiment of the invention, the protein solution is a collagen solution and the cross-linking agent used for hardening is glutaraldehyde. Heat is initially applied to the cell culture microcarriers at 25°C for a period of time between about 1 and 25 hours, preferably, 15 to 20 hours. The heat is then increased to 70°C for a period of time between about 1 and 25 hours, preferably, 6 to 10 hours, more preferably, 8 hours. Lastly, the heat is raised to 100°C and maintained for a period of time from about 1 to 25 hours, preferably, 15 to 20 hours, more preferably, 16 hours. It has been found that when heated over a temperature ramp, such as described above, the cell culture microcarriers harden sufficiently at the initial temperature, without liquifying, so that the temperature may subsequently be raised to accomplish further hardening, again without liquifying the microcarriers.

In those embodiments of the method of the present invention wherein the fluid contains a solidification agent, a solidification agent is present in the solution of the protein or hardening is accomplished by a cross-linking agent, it has been found to be highly preferable, prior to the step of recovering the cell culture microcarriers, to neutralize the excess solidification or cross-linking agent from the cell culture microcarrier.

Neutralization is effected by treating the microcarrier with a dilute solution of the same protein used to form the droplets. Of course, the solution must be sufficiently dilute to avoid solidification of the protein. For a particular protein, one skilled in the art will routinely determine, without undue experimentation, how dilute a neutralizing solution should be to accomplish effective neutralization. In the case wherein collagen solution is used, a dilute collagen solution of about 0.5% by weight may be used to effect neutralization.

In addition to neutralizing, it may also be advantageous to exhaustively wash the cell culture microcarriers, prior to the step of recovering, with saline: Preferably, the microcarriers are washed at least once with saline before and after neutralization.

Further, it may be desired, either before or after the step of recovering the cell culture microcarriers, to sterilize the cell culture microcarriers. Techniques for sterilizing cell culture microcarriers, such as steam sterilization, are well-known.

The cell culture microcarriers of the present invention may be used to grow anchorage-dependent cells. Extensive information on cell culutures is readily available. See e.g., Tissue Culture, Methods and Applications (Ed. Kruse and Patterson) Academic Press, New York, 1973. Moreover, a great deal of information relating to cell culture microcarriers is described in Volumes 46 (1980) and 50 (1982) of Developments in Biological Standardization. The disclosures of all these references are specifically incorporated by reference herein.

The anchorage-dependent cells and/or the anchorage dependent cell products which are grown on the cell culture microcarriers of the present invention may be harvested by any of the conventional, well-known techniques.

For reasons already explained, the cell culture microcarriers of the present invention greatly facilitate subcultivation. Conventional techniques for subcultivation in tissue cultures are well-known. Subcultivation of microcarrier cultures has heretofore been difficult to achieve because, as explained above, it has been difficult to remove the cells from the carriers and thereafter achieve effective cell growth. A preferred technique for subcultivating·in accordance with the present invention involves separating the cell growth from the microcarriers by trypsinization, followed by introducing additional cell culture microcarriers. Alternatively, the cell growth is trypsinized to separate the cells from the microcarriers. The cells are then removed, for example by screening, from the growth medium containing the microcarriers and introduced into a new growth medium. To this new medium, new cell culture microcarriers are added, for example, in double the number of previous microcarriers.

As illustrated in the drawings, the apparatus of the invention for converting water-based protein solution, as defined above, to microspheres, such as cell culture microcarriers, includes a heated thermostatically controlled reservoir for the protein solution. As shown in Figures 1 and 6, the reservoir 20 may be a box-like receptacle having means for maintaining the protein solution 22 at a stable temperature, such as 50°C.

Preferably, the basic supply of protein solution 22 is kept in a first container 24 within the reservoir 20, the container being positioned in a bath 26, containing a heating fluid such as glycerin or water, for maintaining the stable temperature. As shown schematically in Figure 6, a heater 28, controlled by a thermostat 30, supplies heat to the bath 26.

For purposes of rinsing and priming the apparatus, as described hereinafter, the reservoir 20 also includes a second container 32 for holding water 34, the second container also being positioned in the bath 26, for being maintained at the same temperature as the protein solution 22.

In accordance with the invention, the apparatus includes means for supplying the protein solution from the reservoir through at least a substantially horizontal pipe. Preferably, the supplying means includes a loop from and back to the reservoir, the loop including an elongated, substantially horizontal pipe. As embodied herein, the supplying means includes a main liquid communication channel 36 forming a loop including a high pressure pump 38 for circulating the protein solution from the first container 24 through the loop and at least part of the solution back to the first container. For simplicity of illustration, the communication channel 36 in Fig. 1 does not show the complete loop. The complete loop is depicted symbolically, however, in Figure 6.

Preferably, the terminal portion of the communication channel 36 should include flexible members 39a, b, such as hoses. Either or both of the flexible members 39a, b may be selectively moved between the first and second containers 22, 34 for priming or flushing the system with warm water. The apparatus preferably also includes a drain 41, shown symbolically in Figure 6, through which the system can be drained if desired. The drain would, of course, be positioned for accessibility by the flexible member 39a, b.

Preferably also, the container 24 includes a screen or filter 43 for receiving the input end of the communication channel 36 through the flexible member 39a to minimize the possibility of foreign materials entering the communication channel.

The communication channel includes an elongated, substantially horizontal pipe 40, having spaced therealong a plurality of spray heads 42. Only three spray heads 42 are illustrated, but it will be understood that any convenient number of spray heads may be utilized. Advantageously, the spray heads 42 may be placed in series, as is shown in Figs. 1 and 6.

If the high pressure pump 38 is a piston-driven pump, the communication channel 36 should include a buffer 44 and restrictor 46 for attenuating any pulses developed in the circulating protein solution by the pump. Preferably, the buffer 44 and restrictor 46, if utilized, should be located in the communication channel 36 immediately downstream from the pump 38 and certainly between the pump and the spray heads 42. The restrictor 46 may include a filter, if desired (not shown).

The spray heads 42 of the invention are preferably structured as illustrated schematically in Figure 1 and shown in detail in Figs. 3 to 6. As to each spray head 42, the heated protein solution enters the spray head 42 through an inlet port 48 into a cavity 50 of the body 52 of the spray head. The protein solution is forced downward along a barrier 54 into a spray sump 56 and outward through a nozzle 58. Preferably, the nozzle 58 is formed as a disc having a flat upper surface and a circular whirl-plate 60 is mounted in the spray head 42 between the sump 56 and nozzle disc 58.

As shown more particularly in Figs. 4 and 5, the circular whirl-plate 60 is fashioned with at least a pair of canals 62 leading from the sump 56 to a central circular hollow area 64 in the bottom of the whirl-plate and flush against the nozzle disc 58. The canals 62 are symmetrically spaced about the center of the whirl-plate 60 and intersect the hollow area 64 tangentially. As shown in Figure 4, the canals 62 intersect the area 64 on opposite sides of the area. The canals 62 may be L-shaped and formed of a vertical portion 63, such as a hole, and a horizontal portion 65. The horizontal portion 65 may be a groove in the bottom of the whirl-plate 60. This structure induces rotary fluid motion in the hollow area as the protein jet departs the nozzle through a short exit 66. The rotary fluid motion imparted to the jet induces jet breakup at a lower nozzle velocity than would be required without such motion.

Preferably, the sump 56 is provided with a transverse screen 68 of sufficient fineness to prevent materials from clogging the canals 62 of the exit 66. The screen 68 may be clamped between an annular space 70 and an annular gasket 72, the spacer and gasket being locked between the body 52 of the spray head and a threaded nut 74.

If desired, the nozzle disc 58 may have its lower surface machined to form a depression 76 to give strength and rigidity to the disc and to provide outlet space for the exiting spray cone 78 without impingement of the outer surface of the disc.

Also, if desired, the exit 66 may be made noncircular and elongated, instead of circular, for shaping the spray cone 78 as will be described in more detail hereinafter.

Preferably also, the canals 62 are about 0.040" in diameter and the exit 66 in the nozzle disc 58 is about 0.012" long and 0.005" in diameter. This structure of the spray head induces jet instability and breakup of the protein jet into droplets. In addition, it allows droplet formation at relatively low spray pressures and high solution viscosity.

The loop including the diameters of the communication channel 36 and the horizontal pipe 40, together with internal dimensions of the spray heads 42 and the diameters of the canals 62 and exit 66 are such that only a small proportion of the protein solution circulating through the loop passes through the spray heads during one cycle of the protein solution. This design contributes to the stability of temperatures of the protein solution in the spray heads, discussed in more detail hereinafter.

The protein solution not passing through the nozzle 58 is forced upwardly through the cavity 50 on the opposite side of the barrier 54 and outward through the outlet port 79 into the horizontal pipe 40.

Within the barrier wall 54 is formed a thermal sensor well 80 in which is placed any convenient device for monitoring the temperature of the spray head 42. Such a device may be a thermometer for optical monitoring, or a more sophisticated device for registering the temperature in a computerized control.

It is of great importance that the temperature of the spray head remain stable since temperature affects viscosity of the protein solution. Viscosity is an important parameter

0097907

affecting the spray rate delivered by the spray heads and the size distribution of the droplets in the spray. Most preferably, when more than one spray head in series is used, the temperature of the protein solution at the inlet port 48 is the same as the temperature of the protein solution at the outlet port 79.

The apparatus for the invention includes means for applying heat to the pipe for maintaining the protein solution in the spray heads at approximately at least the same temperature as the protein solution in the reservoir. Since excessive heat degrades the protein, it is preferred to store the protein solution at a lower temperature in reservoir 20, but still sufficiently high to maintain a pumpable viscosity, and to heat the protein solution to a higher temperature as the solution approaches a spray head. As embodied herein, the means for applying heat includes heating jackets 84 wrapped around the elongated horizontal pipe 40 in selected positions. Preferably, the heating jackets 84 are positioned upstream of the spray heads.

If desried, heating jackets may also be placed around the communication channel 36 at points remote from the spray heads 42 to maintain the temperature sufficiently high that the solution is maintained at a pumpable viscosity. By use of the heating jackets, predetermined temperatures can be maintained throughout the loop.

In accordance with the invention, the apparatus for converting a water-based protein solution to microspheres includes means for maintaining a predetermined pressure on the protein solution at the spray heads. As embodied herein, the pressure-maintaining means includes an adjustable throttle valve 86 for adjusting the pressure of the protein solution on the spray heads. In addition, the apparatus includes at least one pressure gauge 88 for monitoring the pressure on the loop.

The throttle valve 86 and pressure gauge 88 shown schematically in Fig. 6 may be manually operated and optically observed respectively, or may be more sophisticated devices for complete control of the apparatus of the invention.

The apparatus of the invention also includes at least one receptable positioned beneath a spray head for receiving the droplets emitted by the spray head. Preferably, the number of receptables is equal to the number of spray heads. Preferably, as stated above, there are a plurality of spray heads 42 spaced along the horizontal pipe 40 and a like plurality of receptacles 90 for individually receiving the droplets in the cones 78, emitted by the spray heads.

In accordance with the invention, the apparatus also includes means for flowing a low temperature, water immiscible suspension liquid (fluid liquid medium as described above) across the receptacle for suspending and solidifying the received droplets into discrete microspheres in the liquid suspension. As embodied herein, the flowing means includes a reservoir 92 for containing a supply of the suspension liquid and a pump 94. The pump 94 drives the suspension liquid from the reservoir 92 through a feed pipe 96 having an input at one end of each receptacle 90. The suspension liquid flows downward along the length of the receptacle 90 and into a common drain 98 which empties into a collector 100. A suction pipe 102 pulls the suspension liquid from the bottom of the collector 100 back to the reservoir 92 under the power of the pump 94 completing the loop.

Preferably, as described above, the suspension liquid (liquid fluid medium) is a halogenated hydrocarbon selected from the group consisting of $CH_2Cl_2$, 1-1-1 trichloroethane, trichloroethylene and Freon® T.F.

The reservoir 92 includes means such as a cooling unit for maintaining the low temperature suspension liquid at a temperature of approximately 0° to 10°C, but not below the freezing point of the protein solution. To maintain the low temperature of the suspension liquid, the whole apparatus may be placed in a cold room or the suspension liquid may be passed through a conventional cooler/heat-exchanger, which may be placed in the reservoir 92.

The receptacles 90 are rectangular in shape. Each of the receptacles 90 includes means for developing a pressure head of the suspension liquid across the input section of the receptacle for increasing the speed of flow of the liquid across the receptacle. As embodied herein, the pressure head-developing means is a barrier 104 across the input end of the receptacle and spaced slightly above the bottom of the receptacle. A pressure head of the suspension liquid therefore builds up behind the barrier 104, as shown in Fig. 2, forcing the liquid 106 with increased velocity out from under the barrier 104 and along the bottom of the receptacle 90.

Each receptacle 90 also includes a depressed area 106 at the end opposite the barrier for producing a cascade across the rectangular receptacle 90. The suspension liquid then flows out of each receptacle 90 through an outlet 108 into the common drain 98 and into the collector 100.

The receptacles 90 are preferably dimensioned and positioned for receiving the droplets emanating from the individual spray heads 42 only in the flow of the suspension liquid in the receptacles. As indicated above, the exit apertures 66 of the nozzles 58 may, as shown in Fig. 8, be made non-circular and elongated, if desired, to tend to shape the cones 78, which helps to confine each cone in an individual receptacle 90.

Closable gate means 110, as shown in Fig. 1, may be provided for directly receiving the discharge from one or more spray heads. By such means the spray cone for one or more receptacles may be prevented from entering the receptacles without shutting down the whole apparatus while individual receptacles are being repaired or replaced and also during priming, shutdown and cleaning operation. Closable gate drive means 113, including drive shaft 130, are used to move the closable gate, such as shutters, from the open position, wherein the spray cone discharges into the receptacles, to the closed position, wherein at least one spray cone is prevented from entering the receptacles.

As the droplets in the spray cones fall into the low temperature flow of the liquid suspension, the water-based protein solution is not miscible in the liquid solution. Consequently, the droplets remain integral and solidify in the cold suspension liquid. It is important that the suspension liquid move sufficiently fast to maintain a low steady state concentration of spheres in the liquid surface to prevent still-liquid droplets from impinging on droplets swimming on the surface. Such impinging produces twins and aggregates which are undesirable.

The apparatus of the invention for converting a water-based protein solution to microspheres also includes means for separating the microspheres developed in the receptacles from the liquid suspension. As stated above, the suspension liquid containing the microspheres flows into a common drain 98 as a common discharge. The collector 100 receives the common discharge and the microspheres are filtered therefrom, preferably by screen 112 extending across the collector. Preferably, the screen 112 has a mesh size of about 500 μm and may be removably mounted in the collector 100 by any convenient, known structure.

The invention also includes the method for converting a water-based protein into microspheres comprising spraying the protein solution, described above, as a liquid into a gaseous atmosphere (fluid gaseous medium, as described above) to form discrete droplets, maintaining a temperature differential between the solution and the gaseous atmosphere wherein the gaseous atmosphere is at a lower temperature than the temperature of the solution before spraying, flowing a cooled water immiscible suspension liquid (fluid liquid medium, as described above) across the path of fall of the discrete droplets to form microspheres suspended in the cooled suspension liquid, and collecting the microspheres from the suspension liquid.

The detailed steps of the invention parallel the details of the structure and the functioning of the structure as described hereinbefore.

The following examples are designed to elucidate the teachings of the present invention, and in no way limit the scope of the invention. Various other modifications and equivalents of the examples will readily suggest themselves to those of ordinary skill in the art, particularly after the issuance of this patent, without departing from the spirit or scope of the present invention.

The process described below generally conforms to the schematic diagram shown in Fig. 7.

1. Preparation of solution of denatured, degraded animal collagen.

In a 5 liter container; 440 grams of dry, granular acid-processed (Type A) gelatin (Bloom rating of 250) are dissolved in 3960 ml of distilled water. The gelatine granules are stirred into cold water. Stirring is continued while the temperature is raised to 60°C. Stirring is further continued until a homogeneous viscous solution is obtained. Stirring is then briefly interrupted until bubbles clear out, whereupon stirring is resumed slowly to assure homogeneity.

2. Start-up of protein spray loop.

With reference to Fig. 6, containers 32 and 24 are respectively filled with warm water 34 and a warm protein solution 22, such as the denatured, degraded animal collagen solution, described above, which solution may also contain a slow acting crosslinker, as described above. Thermostat 30 is set for 50°C. The hose flexible members 39a and 39b are placed in hot water container 32. Throttle valve 86 is completely opened. The spray shutter closable gate means 110 are completely closed. Pump 38 is started and hot water is circulated until temperature indicators show steady temperature. The appropriate power setting for heating jackets will either have been determined in previous runs with protein solution or will have to be adjusted in the first run. Hose flexible member 39b is moved to drain 41. Hose flexible member 39a is moved to protein solution 22. As soon as the protein solution has displaced the water in the loop, hose 39b is moved from the drain to the protein container 24. If not already set properly, controls to heating jackets 84 are adjusted until indicators on spray heads 78 show 60°C and the indicator on return 120 shows 50°C. The throttle is now closed until the pressure gauge 88 reaches 170 psi. Of course, spray heads having larger spray patterns will require other pressures, as will be obvious to those of ordinary skill in the art. If necessary, the power to the heating jackets is re-adjusted. These power levels should be noted and kept for later runs. At this stage, sprays of hot protein are discharged onto the spray shutter closable gate means 110. In the described design of Fig. 6 wherein ten spray heads are present, (Fig. 6 shows only three spray heads) the consumption rate of protein solution is 300 ml per minute. An initial amount of approximately 4 liter protein solution, such as the approximately 4 liter collagen solution prepared above, would therefore be consumed in 10 to 15 minutes.

3. Start-up of suspension liquid circulation loop.

With reference to Fig. 1, the pump 94 is started and a flow

of $CH_2Cl_2$ suspension liquid is adjusted to approximately 30 gallons per minute. Valves (not shown in Fig. 1) between the feed pipe 96 and the tray receptacles 90 are once permanently adjusted to give equal flow rate to all trays. At this circulation rate, a 0.25" deep layer of liquid in each of ten 12" wide tray receptacles (only three tray receptacles are shown in Fig. 1) will produce a linear flow rate of 10 cm/sec. A heat-exchanger/cooler (not shown in Fig. 1) is adjusted to give a suspension-liquid temperature between 0.1°C and 10°C in the trays. In a variation, the suspension liquid may contain a diffusible crosslinking agent, as described above. As a further variation, a volatile, diffusible crosslinking agent, as described above, may be contacted with the spray cones prior to contract of the spray droplets with the suspension liquid.

4. Production of sphere suspension in $CH_2Cl_2$.

The spray shutter closable gate means 110 are now opened and droplets of protein solution impinge on the suspension liquid and solidify to discret spheres. The spheres of solidified protein are collected by straining on the screen 112 in collector tank 100. The spheres should have a size distribution of between 250 and 330 μm. The $CH_2Cl_2$ which passes through the screen may be cooled and recycled to the liquid suspension reservoir. After nearly all the protein solution has been consumed, the spray shutters are closed. Pump 94 and the cooling means in the reservoir 92 are shut off. The return hose 39b is moved to drain 41 and the aspirator hose 39a is moved to the hot water container 32. When the circulation loop has been completely flushed with water, all power is shut off.

5. Processing of protein sphere suspension in saline solution.

(a) $CH_2Cl_2$ removal.

Collection tank 100 now contains a cohesive cake of protein microspheres (bead cake) on a coarse screen 112 of 500 μm mesh size.  The screen with the cake is now immersed into a container with saline and the cake is pressed through the screen into the container resulting in a mashed cake in saline.

The screen is now inserted into another empty container and the suspension of spheres is poured into the screen without application of pressure.  The drained liquid contains some $CH_2Cl_2$ and may be discarded.  The cake on the screen is now again pressed into a container with saline and the suspension is stirred with an aerating stirrer.  The shearing effect of the stirrer also helps to reduce the size of aggregates of spheres.  The stirring is continued until no further traces of $CH_2Cl_2$ are expelled.  This operation should be performed in a properly ventilated facility.

   (b)  Removal of air bubbles and released protein.

On settling, the white suspension separates into a floating layer of spheres with air bubbles, while the majority of the protein spheres settles to the bottom.  The clear middle-layer is removed, cold degassed saline is added and after short gentle stirring and settling, this procedure is repeated.  After several repetitions, the suspension becomes clear and all spheres settle to the bottom.   Six liters of packed beads have been obtained in a single run by following this procedure.

   (c)  Screening of beads.

Spheres which are too large or too small are now removed by washing and screening, such as by wet screening.  The oversized and undersized spheres may be dissolved and recycled.  Typically, a screen-size fraction between 260 and 320 μm will be retained.  The volume of the packed retained beads is approximately five liters.  It is important to note that at this point, if no crosslinkers or solification agents have been present in the protein, gas (fluid gaseous medium) or suspension liquid (fluid liquid medium), the spheres and all the protein residue in the pipes, pumps, etc. are easily dissolved in hot water. This aids greatly in housekeeping and particularly in cleaning the screens. Further, it is significant that the processing steps have involved no toxic chemicals other than volatile $CH_2Cl_2$.

6. Diffusion hardening of spheres.

To the suspension of screened spheres in saline, approximately one liter of 25% aqueous glutaraldehyde solution is added as a crosslinking agent for hardening purposes. The suspension is gently stirred at room temeperature for 16 hours. Then, the temperature is raised through a temperature ramp during which only occasional stirring is needed. A typical ramp is 25°C for 16 hours, 70°C for 8 hours and 100°C for 16 hours. This is followed by exhaustive washings with saline until all glutaraldehyde has been removed. As explained above, neutralization is effected with a dilute solution of the collagen.

After neutralization, the spheres may be subjected to a 100°C temperature for about 16 hours and then washed again in saline. The spheres may then be steam sterilized in a conventional fashion. The successive heat-treatments tighten the protein structure and render a steam-sterilizable sphere. At the same time the sphere dimensions contract to about 60% of original size resulting in a volume contraction by a factor of five. By following the procedure described in this example, one liter of amber colored spheres of 150 to 190 µm diameter have been obtained.

Use of 1, 4, butanediol diglycidyl ether or formaldehyde as a crosslinking agent in the procedures described herein has yielded colorless spheres. Addition of the reducing agent sodium dithionite to the glutaraldehyde crosslinking agent has produced spheres which are nearly colorless. However, tests regarding the steam sterilizability of the spheres referred to in this paragraph have not been completed.

7.    Use Of Cell Culture Microcarriers To Grow Diploid
      Human Foreskin Fibroblast Cells

Original cell stock of diploid human fibroblast cells FS-4 is obtained from a roller bottle culture. The cells are removed from the bottle walls by trypsin treatment. To obtain a starter suspension, the content of the bottles is diluted with Dulbecco's modified Eagle's medium plus 5% v/v foetal bovine serum to obtain a cell concentration of $4 \times 10^5 \text{ml}^{-1}$.

To 100 ml of the starter suspension, 10 ml of packed cell culture microcarrier beads are added. The cells and beads (microcarriers) are stirred at 37°C in 10% $CO_2$/90% air atmosphere. The attached cells are counted daily. A typical growth protocol is as follows:

    Day 0 ..... $4 \times 10^5$ cells per ml (starter suspension)
    Day 1 ..... $5,6 \times 10^5$ cells per ml.
    Day 2 ..... $11 \times 10^5$ cells per ml.
    Day 3 ..... $11 \times 10^5$ cells per ml.

On the third day, the microcarrier culture is split for subcultivation. The microcarriers are allowed to settle and the supernatant is decanted. Beads are washed with Eagle's balanced salt solution, each time mixing the beads briefly with one half the original culture volume of solution, allowing the beads to settle and decanting the supernatant.

After washing, the beads are mixed with an equal volume of prewarmed (37°C) trypsin-EDTA solution to initiate cell detachment. The suspension is gently stirred for 10 minutes, at which point all cells have detached.  One half of the bead volume of fetal bovine serum is then added and the microcarriers and cells are centrifuged at 250 G for ten minutes.  The supernatant is discarded.  To the cells and microcarriers, 20 ml of new cell culture microcarriers are added and the suspension is distributed into three spinner vessels bringing the content of the vessels to approximately 100 ml gross medium.  A typical growth protocol for the subcultures is as follows:

Day 3 ..... $3.6 \times 10^5$ cells per ml (first day of split)
Day 4 ..... $5.3 \times 10^5$ cells per ml
Day 5 ..... $11 \times 10^5$ cells per ml.

CLAIMS:

1. A cell culture microcarrier comprising a water-insolubilized protein, said protein being substantially homogeneously distributed throughout said microcarrier.

2. The microcarrier of claim 1, wherein said protein is selected from the group consisting of collagen, casein, soybean protein, silk, keratin, egg proteins and blood proteins.

3. The microcarrier of claim 2, wherein said protein is collagen.

4. The microcarrier of claim 1, wherein said microcarrier comprises the reaction product of said protein and a crosslinking agent.

5. The microcarrier of claim 4, wherein said crosslinking agent is multifunctional.

6. The microcarrier of claim 5, wherein said multifunctional crosslinking agent contains a functional group selected from the group consisting of aldehydes, epoxies, haloalkyls, isothiocyanates, isocyanates, diazo, arylnitrenes, azides, acylhalides, acylanhydrides, diolcarbonates, chloroformates, imidoesters, N-hydroxysuccinimidylesters, maleinimides, carboxydiimidazols, formylesters and halotriazines.

7. The microcarrier of claim 6, wherein said multifunctional crosslinking agent is glutaraldehyde.

8. The microcarrier of claim 6, wherein said multifunctional crosslinking agent is 1, 4, butanediol diglycidyl ether.

9.   The microcarrier of claim 4, wherein said crosslinking agent is selected for the group consisting of formaldehyde, epichlorohydrin, phosgene and aldehyde starch.

10.  The microcarrier of claim 1, wherein said microcarrier has a diameter of about 50 to about 350 microns.

11.  A method for producing cell culture microcarriers comprising the steps of:

     (a) forming droplets of an aqueous solution of a protein;

     (b) suspending said droplets in a fluid;

     (c) solidifying said suspended droplets into cell culture microcarriers;   and

     (d) recovering said cell culture microcarriers.

12.  The method of claim 11, wherein said protein is selected from the group consisting of collagen, casein, soybean protein, silk, keratin, egg proteins and blood proteins.

13.  The method of claim 12, wherein said protein is collagen.

14.  The method of claim 11, wherein said droplets are formed by atomizing said solution from a pressure nozzle.

15.  The method of claim 11, wherein said droplets are formed by atomizing said solution from a gas-propelled spray nozzle.

16.  The method of claim 11, wherein said droplets are formed by atomizing said solution from a rapidly spinning surface.

17.  The method of claim 11, wherein said droplets are formed by atomizing said solution from an ultrasonically driven nozzle.

18. The method of claim 11, wherein said droplets are formed by emulsifying said solution.

19. The method of claim 11, wherein said fluid contains a solidification agent for said droplets.

20. The method of claim 19, wherein said solidification agent is selected from the group consisting of ammonia, an aldehyde, an epoxy compound and epichlorohydrin.

21. The method of claim 20, wherein said aldehyde is a multifunctional aldehyde.

22. The method of claim 21, wherein said multifunctional aldehyde is glutaraldehyde.

23. The method of claim 20, wherein said epoxy compound is a multifunctional epoxy compound.

24. The method of claim 23, wherein said multifunctional epoxy compound is 1, 4 butanediol diglycidyl ether.

25. The method of claim 20, wherein said aldehyde is formaldehyde.

26. The method of claim 11, wherein said fluid is a gaseous medium.

27. The method of claim 26, wherein said gaseous medium is air.

28. The method of claim 26, wherein said gaseous medium contains a compound selected from the group consisting of hydrogen chloride, ammonia, formaldehyde and glutaraldehyde.

29. The method of claim 11, wherein said fluid is a liquid medium.

30. The method of claim 29, wherein said liquid medium is immiscible with said droplets of said solution.

31. The method of claim 30, wherein said liquid medium is a halogenated hydrocarbon.

32. The method of claim 31, wherein said halogenated hydrocarbon is selected from the group consisting of methylene chloride, 1,1,1-trichloroethane, trichloroethylene and Freon® T.F.

33. The method of claim 29, wherein said liquid medium is miscible with said droplets.

34. The method of claim 33, wherein said liquid medium is an acid.

35. The method of claim 34, wherein said acid is an inorganic acid.

36. The method of claim 35, wherein said inorganic acid is selected from the group consisting of nitric acid, hydrochloric acid, phosphoric acid and sulfuric acid.

37. The method of claim 34, wherein said acid is an organic acid.

38. The method of claim 37, wherein said organic acid is selected from the  group consisting of oxalic acid and formic acid.

39. The method of claim 33, wherein said liquid medium is a base.

40. The method of claim 39, wherein said base is selected from the group consisting of ammonia, alkalihydroxide and an amine.

41. The method of claim 33, wherein said liquid medium is an aldehyde.

42. The method of claim 41, wherein said aldehyde is selected from the group consisting of formaldehyde and glutaraldehyde.

43. The method of claim 11, wherein said solidifying is accomplished by maintaining a temperature differential between said droplets of said solution of said protein and said fluid.

44. The method of claim 43, wherein said fluid is hotter than said droplets of said solution.

45. The method of claim 43, wherein said fluid is colder than said droplets of said solution.

46. The method of claim 43, wherein said temperature of said fluid is hotter than the temperature of said droplets of said solution and further, wherein said solution contains a solidification agent.

47. The method of claim 11, wherein said solidifying results from a solidification agent present in said solution of said protein.

48. The method of claim 47, wherein said agent is water soluble and is selected from the group consisting of aldehyde, acid and base.

49. The method of claim 11, further including, after the step of solidifying and prior to the step of recovering, the step of hardening.

50. The method of claim 49, wherein said hardening is accomplished by a crosslinking agent.

51. The method of claim 50, wherein the hardening effect of said crosslinking agent is accelerated by the application of heat.

52. The method of claim 51, wherein the hardening effect of said crosslinking agent is further accelerated by increasing the application of heat without liquifying said cell culture microcarriers.

53. The method of claim 52, wherein said protein is collagen, wherein said crosslinking agent is glutaraldehyde and wherein said heat is initially applied at 25°C for 1 to 25 hours, followed by increasing said heat to 70°C for 1 to 25 hours, followed by increasing said heat to 100°C for 1 to 25 hours.

54. The method of claim 52, wherein said heat is initially applied at 25°C for 15 to 20 hours, followed by applying said heat at 70°C for 6 to 10 hours, followed by applying said heat at 100°C for 15 to 20 hours.

55. The method of claim 50 wherein said crosslinking agent is multifunctional.

56. The method of claim 55, wherein said multifunctional crosslinking agent is selected from the group consisting of aldehyde and epoxy.

57. The method of claim 56, wherein said aldehyde is glutaraldehyde.

58. The method of claim 57, wherein said glutaraldehyde is combined with a reducing agent.

59. The method of claim 58, wherein said reducing agent is sodium dithionite.

60.   The method of claim 50, wherein said crosslinking agent is formaldehyde.

61.   The method of any one of claims 19-25, 47-48 and 50-60, further including, prior to the step of recovering, the  step of neutralizing excess solidification or cross-linking agent from said cell culture microcarrier.

62.   The method of claim 61, wherein said neutralizing is effected by treating said microcarrier with a solution of said protein, said solution being sufficiently dilute to avoid solidification of said protein.

63.   The method of claim 61, further including, after the step of neutralizing and prior to the step of recovering, the step of sterilizing said microcarriers.

64.   The method of claim 61, further including, after the step of recovering, the step of sterilizing said microcarriers.

65.   The method of any one of claims 11 - 18, 26 - 46 and 49, further including, prior to the step of recovering, the step of sterilizing said microcarriers.

66.   The method of any one of claims 11-18, 26-46 and 49, further including, after the step of recovering, the step of sterilizing said microcarriers.

67.   The method of claim 11, wherein said droplets are formed in a gaseous medium and wherein said fluid is a liquid medium.

68.   The method of claim 67, wherein said liquid medium is immiscible with said droplets.

69. A method of growing anchorage-dependent cells comprising the steps of

(a) providing a suspension comprising cell culture microcarriers, an innoculum of said cells and a nutrient-containing growth medium, said microcarriers comprising a water-insolubilized protein, said protein being substantially homogeneously distributed throughout said microcarrier and

(b) maintaining said suspension under conditions conducive to cell growth.

70. A method of producing anchorage-dependent cells comprising the steps of

(a) providing a suspension comprising cell culture microcarriers, an innoculum of said cells and a nutrient-containing growth medium, said cell microcarriers comprising a water-insolubilized protein, said protein being substanially homogeneously distributed throughout said microcarrier,

(b) maintaining said suspension under conditions conducive to cell growth, and

(c) harvesting said cells.

71. A method of producing anchorage-dependent cell products comprising the steps of:

(a) forming a suspension, in a suitable cell culture medium, of cell culture microcarriers comprising a water-insolubilized protein, said protein being substantially homogeneously distributed throughout said microcarrier;

(b) innoculating said culture with anchorage-dependent cells to form a cell culture;

(c)   maintaining said cell culture under conditions conducive to production of cell products;   and

(d)   harvesting said cell products.

72.   The method of claim 71, wherein said cell growth product is interferon.

73.   The method of any one of claims 69, 70 or 71, further including, immediately after the step of maintaining said suspension under conditions conducive to cell growth, the step of  subcultivating said cell growth.

74.   The method of claim 73, wherein said subcultivating is accomplished by separating said cells from said microcarriers and introducing additional cell culture microcarriers into said suspension

75.   The method of claim 73, wherein said subcultivating is accomplished by separating said cells from said microcarriers, removing said cells from said medium and said microcarrier, introducing said cells into a new medium and adding new cell culture microcarriers to said new medium containing said cells.

76.   Apparatus for converting a water-based protein solution to microspheres comprising:

a heated, thermostatically controlled reservoir for the protein solution;

at least one spray head;

means for supplying the protein solution from the reservoir to said spray head;

means for applying heat to said supplying means for maintaining the protein solution in said at least one spray head at approximately at least the same temperature as the protein solution in the reservoir;

means for maintaining a predetermined pressure on the protein solution at the spray head;

at least one receptacle positioned beneath the said spray head for receiving the droplets emitted by the spray head;

means for flowing a low temperature, water immiscible suspension liquid across said at least one receptacle for suspending and solidifying said received droplets into discrete microspheres in said suspension liquid; and

means for collecting said microspheres from said suspension liquid.

77. The apparatus of claim 76 wherein said supplying means includes a loop from and back to said reservoir said loop including an elongated, substantially horizontal pipe, said apparatus includes a plurality of spray heads spaced along said elongated, horizontal pipe and a like plurality of receptacles for individually receiving the droplets emitted by the spray heads and means for maintaining predetermined temperatures throughout said loop.

78. The apparatus of claim 77, wherein said heated reservoir includes a first section for containing the water-based protein solution and a second section for containing water for priming and flushing said circulating means and said spray heads.

79. The apparatus of claim 78, wherein said heated reservoir includes means for maintaining both said first and second sections at a temperature of approximately 50°C.

80. The apparatus of claim 77, wherein said supplying means includes a piston pump for forcing said protein solution through said loop and a buffer and restrictor in said loop for attenuating pulses created by said pump.

81. The apparatus of claim 78, wherein said loop includes a flexible member on the input of said loop and a flexible member on the output of said loop, wherein said flexible member may selectively be inserted either into said first section for circulating said protein solution or into said second section for circulating said heated water.

82. The apparatus of claim 81 also including a drain and wherein either or both of said flexible members may selectively discharge into said drain.

83. The apparatus of claim 81 including a screen in said first section for receiving the input end of said flexible member on the input of said loop.

84. The apparatus of claim 77, wherein each of said spray heads is dimensioned for discharging as atomized solution only a small fraction of the protein solution circulating through said loop.

85. The apparatus of claim 84, wherein each of said spray heads includes means for imparting a swirl to the jet emanating from the spray head.

86. The apparatus of claim 84, wherein each of said spray heads includes means for monitoring the temperature of the spray head.

87. The apparatus of claim 84, wherein each of said spray heads includes a noncircular elongated orifice.

88. The apparatus of claim 77, wherein said heat-applying means includes heating jackets selectively placed on said loop.

89. The apparatus of claim 88 including heating jackets on the upstream sides of said spray heads.

90. The apparatus of claim 77, wherein said pressure maintaining means includes an adjustable throttle for adjusting the pressure of said protein solution on said spray heads.

91. The apparatus of claim 90 including a pressure gauge in said loop.

92. The apparatus of claim 77, wherein said flowing means includes means for circulating said suspension liquid through a loop.

93. The apparatus of claim 92, wherein said suspension liquid circulating means includes a reservoir and a pump.

94. The apparatus of claim 92 also including means for maintaining said low temperature suspension liquid at a temperature of approximately 0° to 10°C but not below the freezing point of the protein solution.

95. The apparatus of any one of claims 77, 92, 93 and 94, wherein each of said receptacles includes means for developing a pressure head of suspension liquid across an input section of each receptacle for increasing the speed of flow of the suspension liquid across the receptacle.

96. The apparatus of claim 95, wherein each of said receptacles is substantially rectangular, said pressure head developing means is a barrier across the input end of the receptacle and spaced slightly above the bottom of the receptacle and each receptacle includes a depressed area at the end opposite said barrier for producing a cascade across the rectangular receptacle.

97. The apparatus of claim 95, wherein said receptacles are dimensioned and positioned for receiving the droplets emanating from the individual spray heads only in the flow of the suspension liquid.

98. The apparatus of claim 77 also including closable gate means for selectively preventing entrance of the discharge from said spray heads into said receptacles.

99. The apparatus of claim 77, wherein said suspension liquid is a halogenated hydrocarbon.

100. The apparatus of claim 99, wherein said halogenated hydrocarbon is selected from the group consisting of $CH_2$ $CL_2$, 1-1-1 trichloroethane, trichloroethylene and Freon® TF.

101. The apparatus of claim 77, wherein said collecting means includes means for separating said spheres from said suspension liquid.

102. The apparatus of claim 96 also including a common discharge for said receptacles and wherein said collecting means includes a filter in said common discharge.

103. The apparatus of claim 102 wherein said filter is a screen having a mesh size of about 500 um.

104. A method for converting a water-based protein solution into microspheres comprising the steps of:

spraying the protein solution as a liquid into a gaseous atmosphere to form discrete droplets;

maintaining a temperature differential between the solution and the gaseous atmosphere wherein the gaseous atmosphere is at a lower temperature than the temperature of the solution before spraying;

flowing a cooled water-immiscible suspension liquid across the path of fall of said discrete droplets to form microspheres suspended in said cooled suspension liquid; and

collecting said cell culture microcarriers from said suspension liquid.

105. The method of claim 104, wherein the step of spraying includes the step of circulating an excess supply of protein solution across a plurality of spray heads.

106. The method of claim 105, wherein the step of circulating a supply of protein solution includes the step of maintaining the circulating protein solution at a temperature of about 50°C.

107. The method of claim 104, wherein said gaseous atmosphere is air.

108. The method of claim 104, wherein the step of flowing the cooled suspension liquid includes the step of maintaining the temperature of the suspension liquid slightly above the freezing temperature of the microspheres.

109. The method of claim 105, wherein the step of flowing includes the step of developing individual flow paths across the fall from the plurality of spray heads.

110. The method of claim 109 including the step of dimensioning the spray cones from the spray heads and the individual flow paths for receiving the spray cones individually and directly onto the flow paths.

111. The method of claim 109, wherein the step of developing individual flow paths includes the step of forming a cascade in each flow path for receiving each spray cone in a cascade.

112. The method of claim 111, wherein the step of forming a cascade includes the steps of raising a pressure head in each flow path and depressing a portion of each flow path downstream from the pressure head.

113. The method of claim 109, wherein the step of collecting the spheres includes the steps of combining the individual flow paths and separating the spheres from the combined flow path.

FIG. 1.

FIG. 2.

FIG. 3.

FIG. 4.

FIG. 5.

FIG. 8.

FIG. 6.

FIG. 7.